# EUROPEAN PATENT SPECIFICATION

(11) **EP 2 380 870 B1**
(45) Date of publication and mention of the grant of the patent: **07.05.2014**
(21) Application number: 10160275.3
(22) Date of filing: 19.04.2010
(51) Int. Cl.: C07C 209/86, C07C 211/05, C01C 1/242

(54) **Method to recover organic tertiary amines from waste sulfuric acid employing a plug flow reactor**
Verfahren zur Wiederherstellung organischer tertiärer Amine aus Abfallschwefelsäure unter Verwendung eines Rohrreaktors
Procédé de récupération d'amines tertiaires organiques à partir de déchets d'acide sulfurique utilisant un réacteur d'écoulement piston

(43) Date of publication of application: 26.10.2011
(73) Proprietor: Celanese International Corporation, Dallas, TX 75234 (US)
(72) Inventor: Brietzke, Stephan, 65624 Altendiez (DE); Groer, Peter, Dr., 63110 Rodgau (DE); Mollenkopf, Carl Christoph, Dr., 65929 Frankfurt am Main (DE)
(74) Representative: Von Kreisler Selting Werner - Partnerschaft von Patentanwälten und Rechstanwälten mbB

(56) References cited:
- WO-A1-2007/079944
- DE-A1- 3 545 196
- DE-C1- 4 416 571

## Description

The invention relates to a method to recover organic tertiary amines from waste sulfuric acid as well as the use of said method to produce ammonium sulfate.

Waste sulfuric acid containing organic tertiary amines is obtained in many chemical plants and is a waste product from various chemical reactions.

The organic tertiary amines do have an economic value and as a consequence, it is desirable to recover the tertiary amines from the waste sulfuric acid. Further, waste sulfuric acid can be converted to ammonium sulfate which is commonly used as a fertilizer. However, it is a requirement for a good inorganic fertilizer that the total amount of organic compounds (TOC) is as low as possible.

It is known that waste sulfuric acid under reclamation of SO₂ can be treated thermally, whereby the contained amines are however lost. The production of SO₂ to the detriment of the contained amines is economically disadvantageous.

DE 101 46 689 A describes a method to recover organic amines from catalysts containing amines by distillative separation.

DE 35 22 470 A describes the recovery of amine- and metallic components in polyphenylene ether synthesis by separation using caustic soda.

DE 44 16 571 describes the recovery of amine from acidic solutions by the addition of alkali bases followed by distillation.

CN 1883790 describes the recovery of amines by neutralization with inorganic bases of oxide origin (NaOH, KOH, Ca(OH)₂, CaCO₃). In so doing the created sulfates must either be disposed of or processed using large amounts of energy (evaporation, drying) in order to obtain a usable by-product. Also, due to the mole masses of the oxide, bases require relatively large mass shares. In case of calcium bases, the created calcium sulfate already precipitates during the reaction and therefore, the suspension must either be diluted or thoroughly blended at substantial costs.

DE 35 45 196 A1 discloses a process to recover tertiary aliphatic amines from waste sulfuric acid with ammonia. However, the yield of the tertiary amines recovered by the process disclosed in DE 35 45 196 A1 is too low and, as a consequence, the total amount of organic compounds which remain in the ammonium sulfate obtained from the process is too high. Thus, the process disclosed in the prior art requires a further purification step in order to reduce the amount of organic compounds in the dry ammonium sulfate to an acceptable level, i.e. a total amount of organic compounds (TOC), preferably of less than 1, more preferably less than 0.5 weight percent based on the dried ammonium sulfate obtained by the process. The TOC is determined in that a sample is oxidized and the amount of generated CO₂ is measured. The TOC can be determined according to the standard method DIN EN 1484-H3. Further, for an acceptable fertilizer it is particular important to keep the amount of organic tertiary amine in the ammonium sulfate composition as low as possible. Further, the process disclosed in DE 35 45 196 A1 does not recover the economically valuable tertiary amines from the waste sulfuric acid in a sufficient amount. Additionally, the process disclosed in DE 35 45 196 A1 is not optimal in terms of energy consumption and use of ammonia. Under the exothermic reaction conditions the added ammonia can evaporate and additionally the energy released by the reaction of waste sulfuric acid with ammonia is not effectively used which, as a consequence, leads to waste of energy.

Therefore, it was an object of the present invention to overcome the problems present in the prior art and in particular, it was an object to significantly increase the yield of tertiary amines recovered from waste sulfuric acid. Further, it was an object to significantly reduce the total amount of organic compounds in the ammonium sulfate obtained from the process to recover the tertiary amine. Additionally, it was an object to reduce the energy consumption of the overall recovery process.

It has surprisingly been found that the problems associated with the methods to recover organic tertiary amines from waste sulfuric acids in the prior art can be solved by a method which uses ammonia which is reacted with waste sulfuric acid in a plug flow reactor.

The invention accordingly provides a method to recover an organic tertiary amine from waste sulfuric acid comprising the following steps:
a) reacting in a plug flow reactor under a pressure ranging from 1.5 to 12 bar
   i) waste sulfuric acid comprising organic tertiary amines with
   ii) ammonia, wherein the ammonia is added in an amount sufficient to obtain a pH of 9.5 or higher; and
b) separating the organic tertiary amine from the reaction mixture obtained in step a), wherein during the separation in step b) the pH of the reaction mixture is adjusted at a pH of 9.5 or higher.

The solution to the above-mentioned problems is surprising since in general to release amines from their ammonium salts, bases need to be used having a basicity of an order of magnitude higher than the corresponding amines. This applies, for example, to the bases described in CN 1883790 or DE-C-44 16 571.

Ammonia, on the other hand, has a comparable or lower basicity than the amines to be reclaimed such as triethylamine and many other amines.

In principle all tertiary amines are suited as organic amines to be recovered from the waste sulfuric acid by the method according to the present invention. The tertiary amines form corresponding hydrogen sulfates (below also referred to as organyl ammonium hydrogen sulfate) with sulfuric acid. Preferred tertiary amines are especially those comprising up to 20 carbon atoms, in particular up to 12 carbon atoms per nitrogen atom. Examples of amines which can be recovered from waste sulfuric acid by the method according to the present invention are selected from the group comprising trimethylamine, triethylamine, diethylpropylamine, tri-n-propylamine, triisopropylamine, ethyldiisopropylamine, tri-n-butylamine, triisobutylamine, tricyclohexylamine, ethyldicyclohexylamine, N,N-dimethylaniline, N,N-diethylaniline, benzyldimethylamine, pyridine, substituted pyridines such as picoline, lutidine, cholidine or methylethylpyridine, N-methylpiperidine, N-ethylpiperidine, N-methylmorpholine, N,N-dimethylpiperazine, 1,5-diazabicyclo[4.3.0]-non-5-en, 1,8-diazabicyclo-[5.4.0]-undec-7-en, 1,4-diazabicyclooctane, tetramethylhexamethylendiamine, tetramethylethylendiamine, tetramethylpropylendiamine, tetramethylbutylendiamine, 1,2-dimorpholylethan, pentamethyldiethyltriamine, pentaethyldiethylentriamine, pentamethyldipropylentriamine, tetramethyldiaminomethane, tetrapropyldiaminomethane, hexamethyltriethylentetramine, hexamethyltripropylenetetramine, diisobutylentriamine and triisopropylentriamine.

Especially preferred is triethylamine.

Ammonia is an inexpensive, easily available chemical basic product and due to its low molecular weight has a highly favorable mass balance.

Ammonia can be used in gaseous or liquid form. According to the invention the partial pressure of the ammonia to be used can be between 0.1 and 300 bar and is limited only by the compressive strength of the used equipment. Ammonia can be used neat or as a mixture with other gases.

Ammonia can be used as a solution in other solvents, preferably as an aqueous solution. The aqueous solution can be obtained commercially or be produced directly from the reaction by introducing gaseous or liquid ammonia in water. The heat of solution can either be removed or retained by transferring the heated solution to the following reaction step. To avoid the exhalation of ammonia it is preferred to work at elevated pressure, e.g. a pressure of higher than 1 bar, preferably 1.5 to 10 bar. In step a) of the method to recover organic tertiary amines from waste sulfuric acid of the present invention, ammonia in gaseous or dissolved form is brought to the reaction with the waste sulfuric acid comprising the organic tertiary amines.

The ammonia is mixed with the waste sulfuric acid in an amount sufficient to obtain a pH of 9.5 or higher. According to a preferred embodiment of the method of the present invention the pH in step a) is preferably ranging from 9.8 to 12, i.e. the ammonia is added to the waste sulfuric acid in an amount sufficient to obtain a pH ranging from 9.8 to 12, more preferably from 10 or higher than 10 to 11.5, especially 10.1 to 11.5.

During the reaction of the waste sulfuric acid with the ammonia, first the free sulfuric acid is neutralized followed by conversion of organyl ammonium hydrogen sulfate to the corresponding amines.

The reaction of the waste sulfuric acid comprising organic tertiary amines with ammonia is conducted in a plug flow reactor at a pressure ranging from 1.5 to 12 bar.

It has surprisingly been found that use of a plug flow reactor in step a) of the method of the present invention is advantageous since the reaction can be conducted under pressure which leads to improved process conditions since the ammonia cannot evaporate under the reaction conditions and, furthermore, the expanded reaction mixture can be introduced into the distillation column which avoids waste of energy since the expansion energy can be used for the distillation of the tertiary amine. In the plug flow reactor the ammonia and the waste sulfuric acid comprising the tertiary amine are continuously introduced and the products are continuously removed, similar to a continuous stirred tank reactor (CSTR). However, rather than randomly mixing the reactants, as in a CSTR, the plug flow reactor provides a substantially even concentration gradient with respect to the residence time in the reactor, after the reactants have been introduced. In an idealized plug flow reactor, the educts are pushed through the tubular vessel, while reacting with each other as they travel, acting as a "plug" or "piston" that travels along the length of a tube. The advantage of the use of a plug flow reactor in the method of the present invention is that the reaction kinetics results in a fast, yet highly efficient conversion of the ammonia with the waste sulfuric acid to the desired products.

In contrast, in the prior art reactions of sulfuric acid with ammonia are usually carried out in a pressure vessel having a huge volume which requires the compliance with complicated safety standards. The plug flow reactor used in the method of the invention, however, has only a small volume and the reaction can be carried out under high pressure and can easily be handled and controlled.

The reaction according to step a) of the method of the present invention is preferably conducted at a pressure ranging from 2 to 12 bar, more preferably from 7 to 10 bar.

The temperature at which the reaction in step a) is conducted is preferably ranging from 95 to 150 °C, more preferably from 100 to 140 °C, most preferably from 110 to 130 °C.

In order to avoid precipitation of ammonium sulfate by exceeding the solubility limit during or after the reaction, water can be added to the reaction mixture. This can be done by diluting the employed waste sulfuric acid with water before the reaction, by adding water during the reaction or by diluting the obtained ammonium sulfate solution after completion of the reaction.

The produced reaction heat can be removed using typical cooling devices known to the person skilled in the art. However, according to a preferred embodiment the released reaction heat of the reaction of step a) is used in separation step b) for the distillative elimination of the organic tertiary amines. Since the reaction in step a) is conducted under elevated pressure in a plug flow reactor the expanded reaction mixture can be directly conveyed to the distillation column. Preferably the method is conducted at temperatures which work at the boiling point of the free amine or if present, the boiling temperature of the amine/water azeotrope or above. In case the reaction heat is not sufficient for distillation an additional heating may be required. For example, in the case of triethylamine the preferred temperature is between 75 and 105 °C at 1 bar.

Further, according to a preferred embodiment of the present method, the energy released in step a) is at least partially used to evaporate the water in the concentration process in order to produce the solid ammonium sulfate, i.e. the reaction heat can be used to evaporate the water from the aqueous ammonium sulfate solution obtained by the method of the present invention.

Preferably an excess of ammonia is mixed with the waste sulfuric acid in order to achieve the preferred pH of 9.5 or higher, more preferably 10.1 or higher.

Solutions which are suitable as waste sulfuric acids contain preferably 0.1 to 100 % by weight of the respective organyl ammonium hydrogen sulfate. Solutions may also contain free sulfuric acid and water. A typical waste sulfuric acid can for example, comprise 35 % by weight triethylammonium hydrogen sulfate, 45 % by weight sulfuric acid, 16 % by weight water and minor amounts of organic components.

In step b) of the method of the present invention the organic tertiary amines are separated from the reaction mixture obtained in step a) wherein during the separation the pH of the reaction mixture is adjusted at a pH of 9.5 or higher. Preferably the pH is adjusted to a range from 9.8 to 12, more preferably from 10, or higher than 10, to 11.5, e.g. 10.1 to 11.5.

The separation of the released amines from the reaction mixture obtained in step a) can be done by distillation, extraction and through phase separation. Distillative separation is especially advantageous for amines with a low boiling point and amines with good water solubility. The above applies especially to amines that form an azeotrope with water. Separation of the organic tertiary amines from the reaction mixture obtained in step a) is preferably conducted in a distillation column.

According to a preferred embodiment the thermal energy of the products obtained at the still head of the distillation column may be used to heat the feed flow, e.g. the ammonia feed or the feed comprising the reaction mixture.

Low solubility amines in ammonium sulfate solution can be obtained through phase separation. Also, the ammonium sulfate solution can be extracted with a suitable solvent. Preferably, the organic tertiary amine is separated from the reaction mixture by extraction with an organic liquid, preferably a liquid hydrocarbon, more preferably an aliphatic liquid hydrocarbon comprising at least 6 carbon atoms, especially octane. However, the type of solvent is only limited by the stability of the used substances, the solubility of the ammonium sulfate solution and the following separability from the extracted amine.

The methods for the separation of the released amines can be applied individually or in combination.

According to an especially preferred method of the present invention the organic tertiary amine, preferably triethylamine, is separated from the reaction mixture obtained in step a) in a distillation column. In order to maintain a pH of 9.5 or higher during the separation in a distillation column, ammonia is preferably added to the distillation column. Preferably during the distillation ammonia is added to the distillation column in a counter flow to the reaction mixture obtained in step a).

According to a preferred embodiment, during distillation the reaction mixture obtained in step a) is continuously fed to the upper part of a distillation column and the ammonia is continuously fed at the lower part or the middle part of the distillation column. The position of the ammonia feed at the distillation column can be used to control the pH of the reaction mixture to be separated during the separation process. The amount of ammonia and consequently the adjusted pH-value influence the capacity of the column with respect to separation of tertiary amines from the aqueous ammonium sulfate solution. The closer the ammonia feed is to the bottom of the distillation column the higher the pH of the reaction mixture in the bottom of the column. The preferred pH value referred to in step b) of the method of the present invention is in case of a separation in a distillation column, the minimum pH value measured in the column between the feed of the reaction mixture and the feed of the ammonia.

Likewise, the position of the ammonia feed at the distillation column also influences the pH value of the aqueous solution comprising ammonium sulfate in the bottom of the column. In a preferred embodiment, the ammonia feed is placed at a position of the distillation column such that the aqueous solution, which is essentially free of the organic tertiary amine, in the lower part of the column has a pH ranging from 5 to 7.

Excess of ammonia can be reintroduced to the process according to the invention. This can be done purposely, e.g. by washing the exhaust containing ammonia with the employed waste sulfuric acid.

According to a preferred embodiment the organic tertiary amine, preferably triethylamine, is recovered in a yield of at least 99.0%, more preferably 99.5%.

According to a further embodiment of the present invention the method of the invention is used to produce ammonium sulfate. The ammonium sulfate solution obtained by the method of the invention represents a quickly recoverable, easily dosable, valuable nitrogen fertilizer. No additional processing is required prior to use. The ammonium sulfate content of the solution can be set as desired by the water content of the used waste sulfuric acid, the addition of water before, during or after the reaction and/or distillative removal of water taking into account the solubility limit of ammonium sulfate in water. Also possible is complete water removal using current methods such as distillation or spray drying, whereby ammonium sulfate is produced as a solid that can be used as a fertilizer.

According to a preferred embodiment, the method of the invention further comprises a dewatering step of the recovered tertiary amine which can optionally be followed by a further distillation of the dewatered amine.

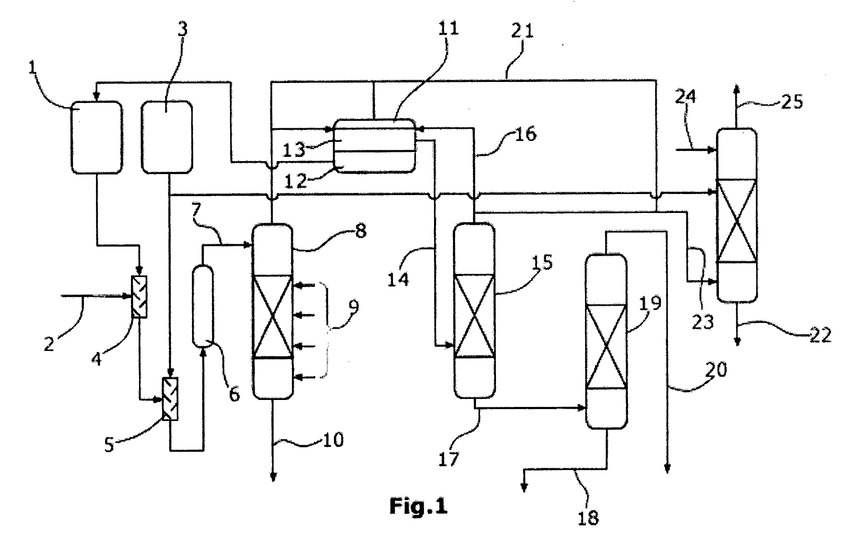

A preferred embodiment of the process of the invention is illustrated by means of the following Figure 1. Figure measures known per se, e.g. addition of stabilizer, are not shown.

Figure 1 shows a method to recover triethylamine from waste sulfuric acid comprising triethylamine.

Figure 1 shows a schematic diagram of a process of the present invention.

### Reference Signs

- 1: water reservoir
- 2: ammonia supply
- 3: waste sulfuric acid reservoir
- 4: first plug flow reactor
- 5: second plug flow reactor
- 6: pipe reactor
- 7: feed for the reaction mixture
- 8: distillation column
- 9: ammonia feed
- 10: flow for the ammonium sulfate solution
- 11: separator
- 12: water phase
- 13: organic phase
- 14: feed for the organic phase
- 15: dewatering column
- 16: feed for an azeotrope of water and tertiary amine
- 17: flow for the tertiary amine
- 18: flow for high boiler
- 19: distillation column
- 20: flow for the purified tertiary amine
- 21: ammonia comprising gases
- 22: flow of washing liquid
- 23: ammonia supply
- 24: water supply
- 25: exhaust gas

Gaseous ammonia is added via ammonia supply (2) to a first plug flow reactor (4) and diluted with water from a water reservoir (1). The aqueous ammonia solution is conveyed to a second plug flow reactor (5) where it is brought into contact with the waste sulfuric acid from the waste sulfuric acid reservoir (3). The waste sulfuric acid and the aqueous ammonia solution are reacted in a pipe reactor (6) and subsequently conveyed to a distillation column (8). The distillation column (8) has different ammonia feeds (9) which can be used to adjust the pH value during the separation process in the column. In the bottom of column (8) the ammonium sulfate solution is obtained which can be released from column (8) by flow (10). The triethylamine-water azeotrope is distilled off and conveyed to the phase separator (11) wherein the azeotrope is separated in a water phase (12) and an organic phase (13) which comprises the triethylamine. The organic phase is fed via feed (14) to the dewatering column (15). The azeotrope of water and triethylamine distilled off in the dewatering column (15) is conveyed via feed (16) to the separator (11). The triethylamine obtained at the bottom of dewatering column (15) is conveyed via flow (17) to distillation column (19). In distillation column (19) the purified triethylamine is distilled off via flow (20). From the bottom of distillation column (19) high boiling organic residues can be separated via flow (18). Ammonia containing gases are conveyed via line (21) and (23) to a column with a flow (22) and a water supply (24) and a supply from the waste sulfuric acid reservoir (3). Exhaust gas which is essentially free from ammonia can be released from the column via line (25).

The ammonia containing washing liquid released via flow (22) can be reintroduced in the process.

### Separation of triethylamine in a distillation column

195 g of ammonia mixed with 755 g of water is reacted with 900 g of waste sulfuric acid consisting of 148 g water, 400 g H₂SO₄ and 334 g triethylammoniumhydrogensulfate (equivalent to 171 g of triethylamine) and additional 700 g of water.

The reaction mixture exhibits a pH value of 10. Subsequently, the reaction mixture is fed to the upper part of a distillation column while at the lower part of the distillation column ammonia is fed to the column in a counter flow fashion in order to keep the pH of the reaction mixture above 10. The pH value of the mixture in the column can be adjusted by the position of the ammonia feed at the lower part of the column.

Different pH values have been adjusted and the amount of triethylamine recovered has been determined. The weight-% of triethylamine recovered is based on the total weight of triethylamine present in the waste sulfuric acid.

**Table 1: Examples 1 and 2 and Comparative Examples 3 to 5**

| Example | pH-value determined during separation | weight-% of triethylamine recovered |
|---|---|---|
| 1 (according to the invention) | 9.5 | 93.0 |
| 2 (according to the invention) | 10.1 | 99.0 |
| 3 (comparative) | 9.0 | 68.0 |
| 4 (comparative) | 7.0 | 2.0 |
| 5 (comparative example 2 of DE 35 45 196) | not mentioned | 92.6% |

## Claims

1. Method to recover an organic tertiary amine from waste sulfuric acid comprising the following steps:
a) reacting in a plug flow reactor at a pressure ranging from 1.5 to 12 bar
i) waste sulfuric acid comprising organic tertiary amines with
ii) ammonia, wherein the ammonia is added in an amount sufficient to obtain a pH of 9.5 or higher; and
b) separating the organic tertiary amine from the reaction mixture obtained in step a), wherein during the separation in step b) the pH of the reaction mixture is adjusted at a pH of 9.5 or higher.

2. Method according to claim 1 wherein the organic tertiary amines are separated from the reaction mixture obtained in step a) in a distillation column.

3. Method according to claim 2 wherein during the distillation ammonia is added to the distillation column.

4. Method according to claim 2 or 3 wherein during the distillation ammonia is added to the distillation column in a counter flow to the reaction mixture obtained in step a).

5. Method according to claim 4 wherein during the distillation the reaction mixture obtained in step a) is continuously fed to the upper part of a distillation column and the ammonia is continuously fed to the lower part or the middle part of the distillation column.

6. Method according to claim 5 wherein the ammonia feed is placed at a position of the distillation column such that the aqueous solution which is free of organic tertiary amine and which comprises the ammonium sulfate in the lower part of the column has a pH ranging from 5 to 7.

7. Method according to at least one of claims 1 to 6 wherein the pH in step a) and/or step b) ranges from 9.8 to 12.

8. Method according to at least one of claims 1 to 7 wherein the organic tertiary amine is triethylamine.

9. Method according to at least one of claims 1 to 8 wherein the organic tertiary amine is recovered in a yield of at least 99.0%.

10. Method according to at least one of claims 1 to 9 wherein the released heat of reaction of step a) is used in separation step b) for distillative elimination of the organic tertiary amine.

11. Method according to at least one of claims 1 to 10 wherein step a) is conducted at a pressure ranging from 2 to 12 bar, more preferably from 7 to 10 bar.

12. Method according to at least one of claims 1 to 11 wherein the reaction in step a) is conducted at a temperature ranging from 90 to 150°C, preferably 100 to 140°C, more preferably from 110 to 130°C.

13. Use of the method according to at least one of claims 1 to 12 to produce ammonium sulfate.

## Patentansprüche

1. Verfahren zur Gewinnung eines organischen tertiären Amins aus Abfallschwefelsäure, umfassend die folgenden Schritte:
a) Umsetzen in einem Kolbenstromreaktor bei einem Druck im Bereich von 1,5 bis 12 bar:
i) Abfallschwefelsäure, die organische tertiäre Amine umfasst, mit
ii) Ammoniak, wobei der Ammoniak in einer ausreichenden Menge hinzugefügt wird, um einen pH-Wert von 9,5 oder mehr zu erhalten; und
b) Abtrennen des organischen tertiären Amins aus dem in Schritt a) erhaltenen Reaktionsgemisch, wobei während des Abtrennens in Schritt b) der pH-Wert des Reaktionsgemischs auf pH 9,5 oder mehr eingestellt wird.

2. Verfahren gemäß Anspruch 1, wobei die organischen tertiären Amine in einer Destillationskolonne aus dem in Schritt a) erhaltenen Reaktionsgemisch abgetrennt werden.

3. Verfahren gemäß Anspruch 2, wobei während der Destillation Ammoniak in die Destillationskolonne gegeben wird.

4. Verfahren gemäß Anspruch 2 oder 3, wobei während der Destillation Ammoniak im Gegenstrom zu dem in Schritt a) erhaltenen Reaktionsgemisch in die Destillationskolonne gegeben wird.

5. Verfahren gemäß Anspruch 4, wobei während der Destillation das in Schritt a) erhaltene Reaktionsgemisch kontinuierlich dem oberen Teil einer Destillationskolonne zugeführt wird und der Ammoniak kontinuierlich dem unteren Teil oder dem mittleren Teil der Destillationskolonne zugeführt wird.

6. Verfahren gemäß Anspruch 5, wobei der Ammoniakzustrom auf einer solchen Position der Destillationskolonne platziert wird, dass die wässrige Lösung, die frei von organischem tertiären Amin ist und das Ammoniumsulfat umfasst, im unteren Teil der Kolonne einen pH-Wert im Bereich von 5 bis 7 aufweist.

7. Verfahren gemäß wenigstens einem der Ansprüche 1 bis 6, wobei der pH-Wert in Schritt a) und/oder Schritt b) im Bereich von 9,8 bis 12 liegt.

8. Verfahren gemäß wenigstens einem der Ansprüche 1 bis 7, wobei es sich bei dem organischen tertiären Amin um Triethylamin handelt.

9. Verfahren gemäß wenigstens einem der Ansprüche 1 bis 8, wobei das organische tertiäre Amin in einer Ausbeute von wenigstens 99,0% gewonnen wird.

10. Verfahren gemäß wenigstens einem der Ansprüche 1 bis 9, wobei die bei der Reaktion von Schritt a) freigesetzte Wärme in Trennschritt b) für die destillative Beseitigung des organischen tertiären Amins verwendet wird.

11. Verfahren gemäß wenigstens einem der Ansprüche 1 bis 10, wobei Schritt a) bei einem Druck im Bereich von 2 bis 12 bar, besonders bevorzugt 7 bis 10 bar, durchgeführt wird.

12. Verfahren gemäß wenigstens einem der Ansprüche 1 bis 11, wobei die Reaktion in Schritt a) bei einer Temperatur im Bereich von 90 bis 150 °C, vorzugsweise 100 bis 140 °C, besonders bevorzugt 110 bis 130 °C durchgeführt wird.

13. Verwendung des Verfahrens gemäß wenigstens einem der Ansprüche 1 bis 12 zur Herstellung von Ammoniumsulfat.

## Revendications

1. Procédé pour récupérer une amine tertiaire organique à partir d'acide sulfurique résiduaire, comprenant les étapes consistant à :
a) faire réagir dans un réacteur à écoulement piston sous une pression comprise entre 1,5 et 12 bar :
i) de l'acide sulfurique résiduaire comprenant des amines tertiaires organiques avec
ii) de l'ammoniac, dans lequel l'ammoniac est ajouté en une quantité suffisante pour obtenir un pH de 9,5 ou plus ; et
b) séparer l'amine tertiaire organique du mélange réactionnel obtenu dans l'étape a), dans lequel le pH du mélange réactionnel est ajusté à un pH de 9,5 ou plus pendant la séparation dans l'étape b).

2. Procédé selon la revendication 1, dans lequel les amines tertiaires organiques sont séparées du mélange réactionnel obtenu dans l'étape a) dans une colonne de distillation.

3. Procédé selon la revendication 2, dans lequel, pendant la distillation, de l'ammoniac est ajouté à la colonne de distillation.

4. Procédé selon la revendication 2 ou 3, dans lequel, pendant la distillation, de l'ammoniac est ajouté à la colonne de distillation à contre courant par rapport au mélange réactionnel obtenu dans l'étape a).

5. Procédé selon la revendication 4, dans lequel, pendant la distillation, le mélange réactionnel obtenu dans l'étape a) est alimenté en continu à la partie supérieure d'une colonne de distillation, et l'ammoniac est alimenté en continu à la partie inférieure ou à la partie moyenne d'une colonne de distillation.

6. Procédé selon la revendication 5, dans lequel l'ammoniac alimenté à la colonne de distillation est positionné de telle manière que la solution aqueuse, qui est exempte d'amine tertiaire organique et qui comprend le sulfate d'ammonium, dans la partie inférieure de la colonne a un pH compris entre 5 et 7.

7. Procédé selon au moins une des revendications 1 à 6, dans lequel le pH dans l'étape a) et/ou dans l'étape b) est compris entre 9,8 et 12.

8. Procédé selon au moins une des revendications 1 à 7, dans lequel l'amine tertiaire organique est la triéthylamine.

9. Procédé selon au moins une des revendications 1 à 8, dans lequel l'amine tertiaire organique est récupérée avec un rendement d'au moins 99,0 %.

10. Procédé selon au moins une des revendications 1 à 9, dans lequel la chaleur de réaction libérée de la réaction dans l'étape a) est utilisée dans l'étape de séparation b) pour l'élimination par distillation de l'amine tertiaire organique.

11. Procédé selon au moins une des revendications 1 à 10, dans lequel l'étape a) est effectuée sous une pression comprise entre 2 et 12 bar, de préférence encore entre 7 et 10 bar.

12. Procédé selon au moins une des revendications 1 à 11, dans lequel la réaction dans l'étape a) est effectuée à une température comprise entre 90 et 150 °C, de préférence entre 100 et 140 °C, de préférence encore entre 110 et 130 °C.

13. Utilisation du procédé selon au moins une des revendications 1 à 12 pour produire du sulfate d'ammonium.
